(19) Europäisches Patentamt
European Patent Office
Office européen des brevets

(11) Numéro de publication : **0 101 379**
**B1**

(12) **FASCICULE DE BREVET EUROPÉEN**

(45) Date de publication du fascicule du brevet : **28.05.86**

(51) Int. Cl.⁴ : **C 07 D307/52**, C 07 D405/12, A 61 K 31/34

(21) Numéro de dépôt : **83401636.2**

(22) Date de dépôt : **10.08.83**

(54) **Benzamides, leurs sels, procédé pour leur préparation et compositions pharmaceutiques les renfermant.**

(30) Priorité : **13.08.82 FR 8214125**

(43) Date de publication de la demande : **22.02.84 Bulletin 84/08**

(45) Mention de la délivrance du brevet : **28.05.86 Bulletin 86/22**

(84) Etats contractants désignés : **AT BE CH DE FR GB IT LI LU NL SE**

(56) Documents cités : **GB-A- 2 065 644**

(73) Titulaire : **SANOFI, société anonyme**
**40, Avenue George V**
**F-75008 Paris (FR)**

(72) Inventeur : **Nisato, Dino**
**Viale Golgi, 80**
**Pavia (IT)**
Inventeur : **Boveri, Sergio**
**Corso Republica 48**
**Tortona (IT)**

(74) Mandataire : **Gillard, Marie-Louise et al**
**Cabinet Beau de Loménie 55, Rue d'Amsterdam**
**F-75008 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

EP 0 101 379 B1

**Description**

La présente invention concerne des uréido, thiouréido, cyanoguanidino et nitroéthènediamino benzamides ayant une activité bloquant les récepteurs $H_2$ de l'histamine, leurs sels, un procédé pour leur préparation et des compositions pharmaceutiques les renfermant en tant qu'ingrédients actifs.

La demande de brevet français publiée sous le numéro 2 471 376 décrit et revendique des composés ayant la formule

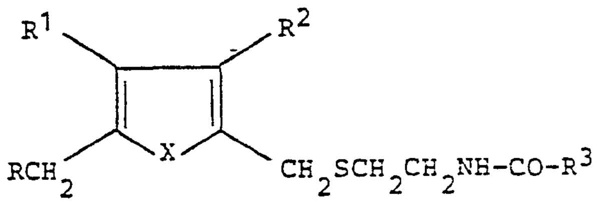

(I)

où R est le groupe diméthylamino ou 1-pyrrolidinyle ; $P^1$ et $P^2$ représentent chacun l'hydrogène ou un groupe alkyle en C-$C_3$ ; $R^3$ est l'hydrogène, un groupe alkyle en $C_1$-$C_3$ (substitué de manière facultative par un membre choisi dans le groupe se composant de groupes cyano, alkoxy en $C_1$-$C_3$, phényle, et d'un groupe hétérocyclique pentagonal ou hexagonal), un groupe cycloalkyle en $C_3$-$C_6$, un groupe alkényle en $C_2$-$C_5$ (substitué de manière facultative par un membre choisi dans le groupe se composant de groupes alkoxy en $C_1$-$C_3$, phényle et phénoxy), un groupe aryle en $C_6$-$C_{10}$ (substitué de manière facultative par un ou deux membres choisis dans le groupe se composant de groupes hydroxy, halogène, nitro, sulfamoyle, alkyle en $C_1$-$C_3$, alkoxy en $C_1$-$C_3$, alkanoyle en $C_1$-$C_3$, alkoxycarbonyle en $C_2$-$C_4$, dialkylamino en $C_2$-$C_4$ et alkanesulfonyle en $C_1$-$C_3$), ou un groupe hétérocyclique pentagonal ou hexagonal (éventuellement substitué par un membre choisi dans le groupe se composant des groupes oxo, halogène, alkyle en $C_1$-$C_3$ et alkoxy en $C_1$-$C_3$) et X est l'oxygène ou le soufre, ainsi que leurs sels d'addition avec les acides pharmaceutiquement acceptables.

Parmi les produits décrits dans la demande de brevet ci-dessus, le composé de formule I où R = diméthylamino, $R^1$ = $R^2$ = H et $R^3$ = 4-sulfamoylphényle, à savoir la N-[2-(5-diméthylaminométhylfuran-2-ylméthylthio)éthyl]-4-sulfamoylbenzamide, sous forme d'oxalate, montre une valeur de DE50 de 2,54 mg/kg dans l'activité d'inhibition de la sécrétion d'acide de l'estomac chez le rat.

La demande de brevet français ci-dessus ne comprend dans sa formule générale aucun benzamide substitué dans le noyau benzénique par un groupe uréido, thiouréido, guanidino ou nitroéthènediamino.

On a maintenant trouvé que de nouveaux benzamides, présentant un groupe uréido, thiouréido, guanidino ou nitroéthènediamino dans la position méta du noyau benzénique, possèdent une bonne action antagonisant les récepteurs $H_2$ de l'histamine.

Ainsi la présente invention, selon un de ses aspects, a pour objet des benzamides de formule

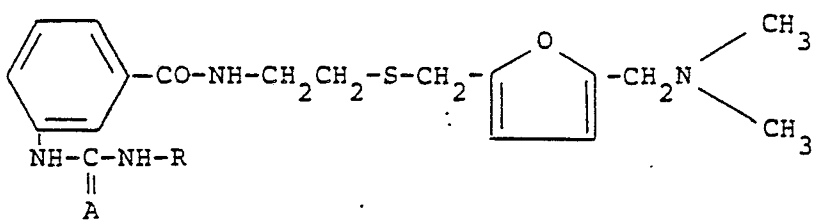

(II)

dans laquelle A représente un atome d'oxygène ou de soufre, un groupe N—CN ou CH—$NO_2$ et R représente un groupe alkyle contenant de 1 à 6 atomes de carbone, un groupe phényle, un groupe pyridyle ou un groupe pyridyle 1-oxyde, éventuellement substitués, ainsi que leurs sels pharmaceutiquement acceptables.

Les sels pharmaceutiquement acceptables comprennent les sels non toxiques dérivés d'acides minéraux ou organiques tels que le chlorhydrate, le bromhydrate, le sulfate, le succinate, le tartrate, le citrate, le fumarate, le maléate, le 4,4'-méthylènebis-(3-hydroxy-2-naphtoate), ci-après désigné « pamoate », le 2-naphtalènesulfonate, ci-après désigné « napsylate », le méthanesulfonate, ci-après désigné « mésylate », le p-toluènesulfonate, ci-après désigné « tosylate », et similaires.

Selon un autre de ses aspects, la présente invention a pour objet un procédé pour la préparation des composés de formule II ci-dessus, ledit procédé étant caractérisé en ce que l'on traite le 3-amino-N-[2-(5-diméthylaminométhylfuran-2-ylméthylthio)éthyl]benzamide de formule

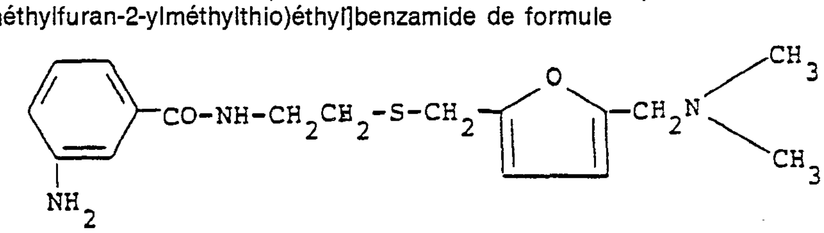

(III)

avec un composé de formule

$$Y = C = Z \qquad (IV)$$

dans laquelle Y représente deux groupes méthylthio et Z représente un groupe N—CN ou CH—$NO_2$, ou bien Y représente un atome d'oxygène ou de soufre et Z représente un groupe N—R, où R a la même signification donnée ci-dessus, dans un solvant organique à une température comprise entre 20 °C et la température d'ébullition du solvant employé et, lorsque Y représente deux groupes méthylthio et Z représente un groupe N—CN ou CH—$NO_2$, on traite le produit résultant avec une amine de formule R—$NH_2$, où R est tel que défini ci-dessus, dans un solvant organique ; et on transforme éventuellement le produit ainsi obtenu dans ses sels pharmaceutiquement acceptables.

Comme solvant de réaction on utilise de préférence un alcool, tel que le méthanol, l'éthanol ou l'isopropanol, l'éthanol étant particulièrement préféré.

Selon un autre de ses aspects, la présente invention concerne, en tant que produit intermédiaire, le 3-amino-N-[2-(5-diméthylaminométhylfuran-2-ylméthylthio)éthyl]benzamide de formule III ci-dessus ainsi que ses sels d'addition.

Ledit composé, qui n'est pas décrit en littérature et qui n'est pas compris dans la demande de brevet français n° 2 471 376, représente l'intermédiaire clé pour le procédé de la présente invention, mais il est également utile pour la préparation des amidobenzamides décrits dans la demande de brevet européen publiée sous le n° 69 664.

En fait, on a trouvé qu'à partir de la nouvelle amine III on peut préparer, par simple réaction avec un dérivé fonctionnel d'un acide carboxylique ou sulfonique, toute la série des amidobenzamides décrite dans la demande de brevet européen ci-dessus. On évite ainsi de devoir préparer, pour chaque synthèse, des intermédiaires particuliers.

Le 3-amino-N-[2-(5-diméthylaminométhylfuran-2-ylméthylthio)étyl]benzamide peut être préparé par réduction du composé de formule

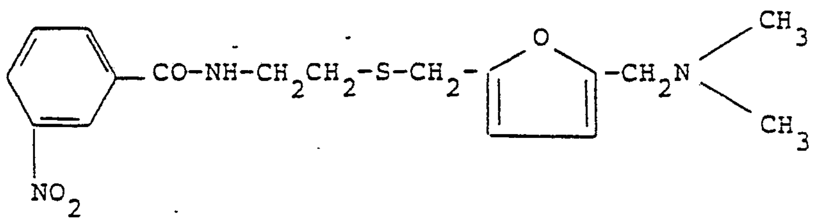

(V)

en utilisant, comme agent réducteur, l'hydrogène naissant de la réaction entre un métal, tel que le fer, et l'acide chlorhydrique.

Le produit est isolé selon les méthodes conventionnelles sous forme d'une huile assez instable à la chaleur et même à la température ambiante, qui doit être conservé au frais. Sous forme de sel, il peut être conservé sans problèmes ; l'oxalate est particulièrement préféré.

De toute façon, le 3-amino-N-[2-(5-diméthylaminométhylfuran-2-ylméthylthio)éthyl]benzamide base libre que l'on obtient à la fin de la réaction est suffisamment pure pour sa transformation dans les benzamides de la présente invention.

Le composé de départ V, à savoir le 3-nitro-N-[2-(5-diméthylaminométhylfuran-2-ylméthylthio)éthyl]benzamide, est également un produit nouveau qui peut être aisément préparé selon le procédé décrit dans la demande de brevet français publiée au n° 2 471 376 citée ci-dessus.

Les benzamides de formule II ci-dessus, ainsi que leurs sels pharmaceutiquement acceptables, agissent comme antagonistes sélectifs des récepteurs $H_2$ de l'histamine et sont donc utiles pour le traitement de la maladie ulcéreuse.

La sélectivité de l'activité des produits de la présente invention vers les récepteurs du type $H_2$ est confirmée par l'absence d'activité du type $H_1$ dans le test de la contraction provoquée par l'histamine sur l'iléon isolé de cobaye.

L'activité antagoniste des composés de la présente invention vers les récepteurs $H_2$ gastriques de l'histamine a été confirmée dans le test de l'activité antisécrétoire basé sur l'antagonisme pour l'hypersécrétion provoquée par l'histamine chez le rat atropinisé, selon la méthode de Ghosh et Schild (Br. J. Pharmacol. Chemother. 1958, 13, 54) modifiée selon Plack (Nature 1972, 236, 385). Selon ce test, on provoque une hypersécrétion acide gastrique par infusion intraveineuse d'une dose submaximale d'histamine équivalent à 15 mcmol/kg/heure et l'on mesure la sécrétion gastrique par perfusion d'une solution physiologique à une vitesse constante dans l'estomac de l'animal.

Le tableau I montre, pour quatre composés représentatifs de la présente invention, désignés par leurs codes SR 57914, SR 57934A, SR 57970 et SR 58028A et pour trois produits de référence, la 2-cyano-1-méthyl-3-[2[[(5-méthylimidazol-4-yl)méthyl]thio]éthyl]guanidine, ci-après désignée par sa Dénomination Commune Internationale « cimétidine », la N-[2-[[5-[(diméthylamino)méthyl]furfuryl]thio]éthyl]-N'-méthyl-2-nitro-1,1-éthènediamine, ci-après désignée par sa Dénomination Commune Internationale « ranitidine » et le N-[2-(5-diméthylaminométhylfuran-2-ylméthylthio)éthyl]-4-sulfamoylbenzamide, décrit

dans la demande de brevet français 2 471 376 citée ci-dessus, ci-après désigné « Composé A », la dose (en mcmol/kg par voie veineuse en dose unique) qui inhibe de 50 % l'hypersécrétion gastrique provoquée par l'histamine (DI50), ainsi que la puissance relative de chaque produit par rapport à la cimétidine. La DI50 représente l'index de l'activité $H_2$-bloquante gastrique.

Tableau I

| Composé | DI50 (mcmol/kg) | Puissance relative (cimétidine = 1) |
|---|---|---|
| Cimétidine | 0,95 | 1,00 |
| Ranitidine | 0,25 | 3,80 |
| Composé A | 2,26 | 0,42 |
| SR 57914 | 0,45 | 2,11 |
| SR 57934 A | 1,53 | 0,62 |
| SR 57970 | 0,50 | 1,90 |
| SR 58028 A | 0,23 | 4,13 |

De ce tableau il ressort que les composés représentatifs de la présente invention sont tous plus actifs que le Composé A ; leur activité est comparable ou supérieure à celle de la cimétidine et un d'entre eux est au moins aussi actif que la ranitidine.

L'activité antisécrétoire des produits de la présente invention a été évaluée chez le chat porteur de fistule gastrique selon la méthode de Emas et al. (Gastroenterology, 1960, 39, 771) en utilisant, comme hypersécréteur, le dimaprit à la dose de 640 mcg/kg/h. Dans ces conditions, un composé représentatif de la présente invention, le SR 57914, administré par voie intragastrique une heure avant la perfusion du dimaprit, antagonise de manière dose-dépendante l'hypersécrétion provoquée par le dimaprit. Son activité est au moins égale à celle de la cimétidine utilisée comme composé de référence.

Les composés de la présente invention sont peu toxiques et sont utiles comme médicaments.

Ainsi, selon un autre de ses aspects, la présente invention se réfère à des compositions pharmaceutiques renfermant, en tant qu'ingrédients actifs, les composés de formule II ci-dessus, ainsi que leurs sels d'addition pharmaceutiquement acceptables.

Dans les compositions pharmaceutiques de la présente invention pour l'administration orale, sublinguale, sous-cutanée, intramusculaire, intraveineuse, transdermique ou rectale, les ingrédients actifs de formule II ci-dessus peuvent être administrés sous des formes unitaires d'administration, en mélange avec des supports pharmaceutiques classiques, aux animaux et aux êtres humains pour le traitement de l'hypersécrétion gastrique et de la maladie ulcéreuse. Les formes unitaires d'administration appropriées comprennent les formes par voie orale telles que les comprimés, les gélules, les poudres, les granulés et les solutions ou suspensions orales et les formes d'administration sublinguale et buccale, les formes d'administration parentérale utiles pour une injection sous-cutanée, intramusculaire ou intraveineuse, de même que les formes pour l'administration rectale.

Afin d'obtenir l'effet antisécrétoire désiré, la dose de principe actif peut varier entre 0,1 et 100 mg par kg de poids du corps et par jour.

Chaque dose unitaire peut contenir de 10 à 1000 mg, de 50 à 500 mg de préférence, d'ingrédient actif en combinaison avec un support pharmaceutique. Cette dose unitaire peut être administrée 1 à 4 fois par jour pour traiter l'hypersécrétion gastrique et la maladie ulcéreuse.

Les exemples suivants illustrent l'invention sans toutefois la limiter.

Exemple 1

a) 3-nitro-N-[2-(5-diméthylaminométhylfuran-2-ylméthylthio)éthyl]benzamide.

A une solution de 10,7 g de 2-(5-diméthylaminométhylfuran-2-ylméthylthio)éthylamine et de 5,86 g de 4-diméthylaminopyridine dans 100 ml de chlorure de méthylène, on ajoute, goutte à goutte, sous agitation et à la température de — 5 à 0 °C, une solution de 9,5 g de chlorure de 3-nitrobenzoyle dans 50 ml de chlorure de méthylène, puis on agite le mélange réactionnel 30 minutes à 0 °C et, ensuite, 2 heures à la température ambiante. On évapore à sec sous pression réduite, on reprend le résidu avec 100 ml d'acide chlorhydrique N, on lave la solution acide deux fois avec 30 ml d'acétate d'éthyle et l'on ajuste son pH à 7,8. On extrait avec de l'acétate d'éthyle contenant 10 % d'éthanol, on sèche la solution organique sur du sulfate de sodium anhydre et on l'évapore. On obtient 16 g de base libre sous forme d'une huile jaune pâle.

On dissout l'huile ainsi obtenue dans de l'isopropanol et on traite la solution résultante avec de l'acide oxalique en isopropanol. On obtient ainsi 17,8 g d'oxalate de 3-nitro-N-[2-(5-diméthylaminométhylfuran-2-ylméthylthio)-éthyl]benzamide ; p. f. 146-148 °C.

b) 3-amino-N-[2-(5-diméthylaminométhylfuran-2-ylméthylthio)éthyl]benzamide.

A une suspension de 13 g de 3-nitro-N-[2-(5-diméthylaminométhylfuran-2-ylméthylthio)éthyl]benzamide et 8 g de fer métallique, lavé au préalable avec de l'acide chlorhydrique 2 N, dans un mélange de 50 ml d'eau et de 50 ml de méthanol, on ajoute, goutte à goutte, sous agitation, de l'acide chlorhydrique concentré jusqu'à pH 5, puis on chauffe le mélange 2 heures au reflux. Après refroidissement, on filtre et on lave à fond le précipité avec du méthanol. La solution ainsi obtenue est ensuite évaporée sous pression réduite pour éliminer le méthanol. On ajuste le pH de la solution aqueuse à 10 avec de l'hydroxyde de sodium et on élimine le précipité inorganique qui s'est formé, après l'avoir lavé à l'éthanol. On extrait la solution 4 fois avec 40 ml d'acétate d'éthyle contenant 10 % d'éthanol, on sèche la solution dans l'acétate d'éthyle sur du sulfate de sodium anhydre et on l'évapore à sec. On obtient 9 g de 3-amino-N-[2-(5-diméthylaminométhylfuran-2-ylméthylthio)éthyl]benzamide base sous forme d'une huile.

On dissout le produit ainsi obtenu dans de l'éthanol et on traite la solution alcoolique avec de l'acide oxalique dans de l'éthanol à 95 %. On obtient ainsi 13 g d'oxalate de 3-amino-N-[2-(5-diméthylaminométhylfuran-2-ylméthylthio)-éthyl]benzamide ; p. f. 112-115 °C.

## Exemple 2

A une solution de 6 g de 3-amino-N-[2-(5-diméthylaminométhylfuran-2-ylméthylthio)éthyl]benzamide dans 50 ml d'éthanol absolu, on ajoute, goutte à goutte, une solution de 2,3 g d'isocyanate de méthyle dans 10 ml d'éthanol absolu, puis on abandonne le mélange 30 minutes à la température ambiante et on la chauffe ensuite à 50 °C pendant 2 heures. On évapore le solvant sous pression réduite, on reprend le résidu avec de l'acide chlorhydrique dilué, on extrait la solution aqueuse deux fois avec 100 ml d'acétate d'éthyle et on la traite avec de l'hydroxyde de sodium concentré jusqu'à pH nettement basique. On extrait trois fois avec 100 ml d'acétate d'éthyle, on lave à l'eau la phase organique, on la sèche sur du sulfate de sodium anhydre et on l'évapore à sec. On obtient ainsi le 3-(3-méthyluréido)-N-[2-(5-diméthylaminométhylfuran-2-ylméthylthio)éthyl]benzamide sous forme d'une huile qui est dissoute dans l'isopropanol et traitée avec une solution de 1,5 g d'acide oxalique dans l'isopropanol. Après cristallisation dans l'éthanol 95 %, on obtient 4,5 g d'oxalate de 3-(3-méthyluréido)-N-[2-(5-diméthylaminométhylfuran-2-ylméthylthio)éthyl]benzamide sous forme d'un solide blanc ; p. f. 156-158 °C. Numéro de code : SR 57934A.

De la même façon, par réaction de 10 g de 3-amino-N-[2-(5-diméthylaminométhylfuran-2-ylméthylthio)éthyl]benzamide dans 50 ml d'éthanol avec 9 g d'isocyanate de phényle, on obtient, par cristallisation dans l'acétate d'éthyle, 7,5 g de 3-(3-phényluréido)-N-[2-(5-diméthylaminométhylfuran-2-ylméthylthio)éthyl]benzamide ; p. f. 133-135 °C. Numéro de code : SR 57972.

De même, à partir de 5 g de 3-amino-N-[2-(5-diméthylaminométhylfuran-2-ylméthylthio)éthyl]benzamide et 1,4 g d'isothiocyanate de méthyle dans 60 ml d'éthanol, on obtient, par cristallisation dans l'éthanol 95 %, 2,5 g de 3-(3-méthylthiouréido)-N-[2-(5-diméthylaminométhylfuran-2-ylméthylthio)éthyl]benzamide ; p. f. 155-157 °C. Numéro de code : SR 57914.

## Exemple 3

On chauffe au reflux pendant 30 heures un mélange de 20 g de 3-amino-N-[2-(5-diméthylaminométhylfuran-2-ylméthylthio)-éthyl]benzamide et 8,8 g de cyanodithioiminocarbonate de diméthyle dans 150 ml d'éthanol absolu, puis on concentre sous pression réduite et on pose l'huile brute obtenue sur une colonne de silice en éluant avec un mélange chloroforme : méthanol 4 : 1. On réunit les fractions suffisamment pures et l'on évapore le solvant sous pression réduite. On obtient 6,5 g de 3-(3-cyano-2-méthylisothiouréido)-N-[2-(5-diméthylaminométhylfuran-2-ylméthylthio)éthyl]benzamide sous forme d'une huile qui est mélangée à 10 ml d'une solution à 33 % de méthylamine en éthanol. Le mélange ainsi obtenu est abandonné 48 heures à la température ambiante, puis on élimine l'excès de méthylamine sous pression réduite. On obtient 5 g d'une huile qui est soumise à une chromatographie flash sur silice en éluant avec un mélange chloroforme : méthanol 1 : 1. On évapore les fractions pures réunies et l'on cristallise le résidu deux fois dans l'isopropanol. On obtient 2,5 g de 3-(2-cyano-3-méthylguanidino)-N-[2-(5-diméthylaminométhylfuran-2-ylméthylthio)-éthyl]benzamide ; p. f. 140-142 °C (SR 57970).

Selon la procédure décrite ci-dessus, par réaction de 0,02 mol de 3-amino-N-[2-(5-diméthylaminométhylfuran-2-ylméthylthio)éthyl]benzamide et 0,02 mol de 1,1-bis-méthylthio-2-nitroéthène dans 50 ml d'éthanol on obtient, avec un rendement de 65 %, le 3-[N-(1-méthylthio-2-nitroéthényl)amino]-N-[2-(5-diméthylaminométhylfuran-2-ylméthylthio)éthyl]benzamide qui, dissout dans de l'éthanol et traité avec une solution à 33 % de méthylamine dans de l'éthanol donne le 3-[N-(1-méthylamino-2-nitroéthényl)-amino]-N-[2-(5-diméthylaminométhylfuran-2-ylméthylthio)-éthyl]benzamide sous forme d'une huile pratiquement pure. On transforme le produit ainsi obtenu dans son oxalate dans de l'éthanol à 95 %. Après cristallisation dans 200 ml d'éthanol, on obtient l'oxalate pur ; p. f. 175-177 °C. Rendement : 43 % (SR 58 028A).

Exemple 4

Comprimés à base d'un des composés décrits dans les Exemples 2 et 3, ayant la composition suivante :

| | |
|---|---|
| principe actif | 150 mg |
| cellulose microcristalline | 75 mg |
| talc | 15 mg |
| polyvinylpyrrolidone | 30 mg |
| silice précipitée | 25 mg |
| stéarate de magnésium | 5 mg |

On mélange intimement dans une mélangeuse-pétrisseuse tous les ingrédients, sauf le lubrifiant, pendant 15 minutes, puis on pétrit par addition graduelle d'eau. On fait passer la masse à travers un tamis de 1,25 mm. On sèche le granulé dans une étuve à ventilation forcée jusqu'à ce qu'on obtient une humidité résiduelle relativement réduite (environ 2 %). On uniformise le granulé, on ajoute le lubrifiant et l'on forme des comprimés par compression. Poids d'un comprimé : 300 mg.

De la même façon on prépare des comprimés contenant 250 mg de principe actif.

Exemple 5

Gélules contenant un des composés décrits dans les Exemples 2 et 3, ayant la composition suivante :

| | |
|---|---|
| principe actif | 200 mg |
| amidon de mais | 90 mg |
| talc | 10 mg |

On mélange intimement le principe actif et les excipients et l'on introduit le mélange ainsi obtenu dans des gélules de gélatine de dimension 1. Contenu d'une gélule : 300 mg.

**Revendications** (pour les Etats contractants : BE, CH, DE, FR, GB, IT, LI, LU, NL, SE)

1. Benzamides de formule

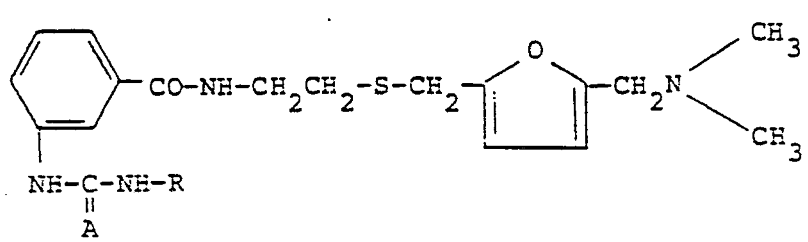

dans laquelle A représente un atome d'oxygène ou de soufre, un groupe N—CN ou CH—$NO_2$ et R représente un groupe alkyle contenant de 1 à 6 atomes de carbone, un groupe phényle, un groupe pyridyle ou un groupe pyridyle 1-oxyde, éventuellement substitués ; ainsi que leurs sels pharmaceutiquement acceptables.

2. 3-(3-méthylthiouréido)-N-[2-(5-diméthylaminométhylfuran-2-ylméthylthio)éthyl]benzamide et ses sels pharmaceutiquement acceptables.

3. Procédé pour la préparation d'un composé de formule

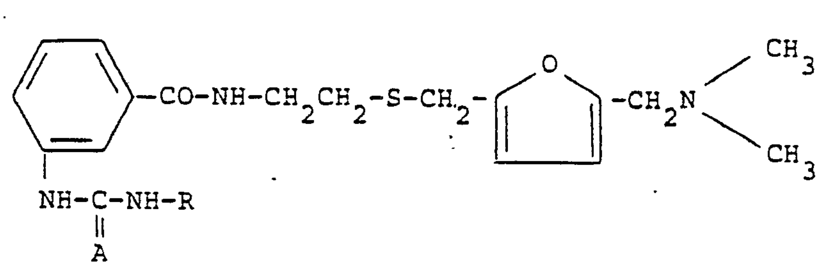

dans laquelle A représente un atome d'oxygène ou de soufre, un groupe N—CN ou CH—$NO_2$ et R représente un groupe alkyle contenant de 1 à 6 atomes de carbone, un groupe phényle, un groupe pyridyle ou un groupe pyridyle 1-oxyde, éventuellement substitués et de ses sels pharmaceutiquement

acceptables, caractérisé en ce que l'on traite le 3-amino-N-[2-(5-diméthylaminométhylfuran-2-ylméthylthio)éthyl]benzamide de formule

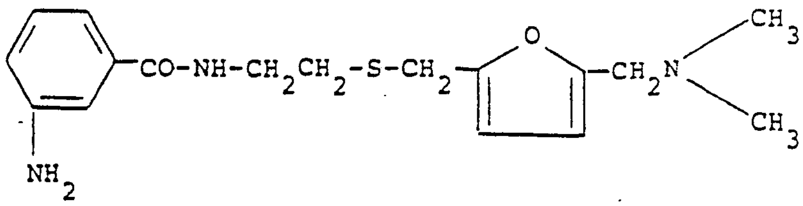

avec un composé de formule

Y = C = Z

dans laquelle Y représente deux groupes méthylthio et Z représente un groupe N—CN ou CH—$NO_2$, ou bien Y représente un atome d'oxygène ou de soufre et Z représente un groupe N—R, où R a la même signification donnée ci-dessus, dans un solvant organique à une température comprise entre 20 °C et la température d'ébullition du solvant employé et, lorsque Y représente deux groupes méthylthio et Z représente un groupe N—CN ou CH—$NO_2$, on traite le produit résultant avec une amine de formule R—$NH_2$, où R est tel que défini ci-dessus, dans un solvant organique ; et on transforme éventuellement le produit ainsi obtenu dans ses sels pharmaceutiquement acceptables.

4. Procédé selon la revendication 3, caractérisé en ce que le produit de départ, 3-amino-N-[2-(5-diméthylaminométhylfuran-2-ylméthylthio)éthyl]benzamide, ou un de ses sels, est préparé par réduction du 3-nitro-N-[2-(5-diméthylaminométhylfuran-2-ylméthylthio)éthyl]benzamide en utilisant, comme agent réducteur, l'hydrogène naissant de la réaction entre fer et acide chlorhydrique.

5. 3-amino-N-[2-(5-diméthylaminométhylfuran-2-ylméthylthio)éthyl]benzamide de formule

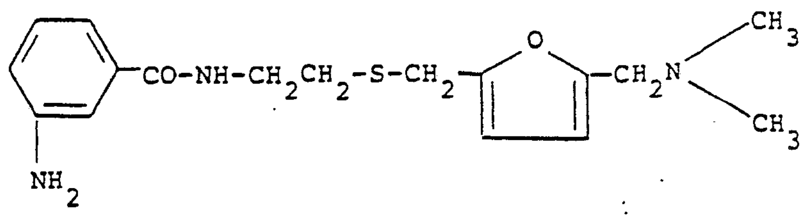

et ses sels d'addition acides.

6. Composition pharmaceutique renfermant, en tant que principe actif, un composé selon une des revendications 1 et 2.

7. Composition pharmaceutique selon la revendication 6, sous forme d'unité de dosage, dans laquelle chaque unité de dosage contient de 10 à 1000 mg d'ingrédient actif en mélange avec un excipient pharmaceutique.

8. Composition pharmaceutique selon la revendication 7, contenant de 100 à 500 mg d'ingrédient actif par unité de dosage.

**Revendications** (pour l'Etat contractant AT)

1. Procédé pour la préparation d'un composé de formule

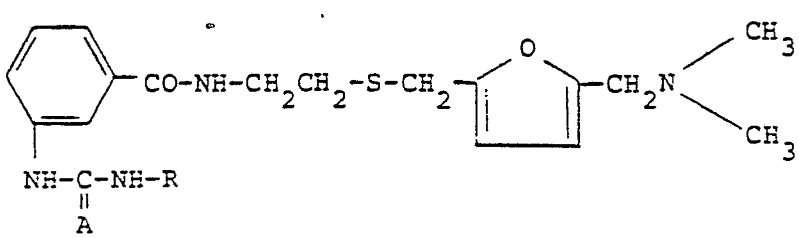

dans laquelle A représente un atome d'oxygène ou de soufre, un groupe N—CN ou CH—$NO_2$ et R représente un groupe alkyle contenant de 1 à 6 atomes de carbone, un groupe phényle, un groupe pyridyle ou un groupe pyridyle 1-oxyde, éventuellement substitués et de ses sels pharmaceutiquement acceptables, caractérisé en ce que l'on traite le 3-amino-N-[2-(5-diméthylaminométhylfuran-2-ylméthylthio)éthyl]benzamide de formule

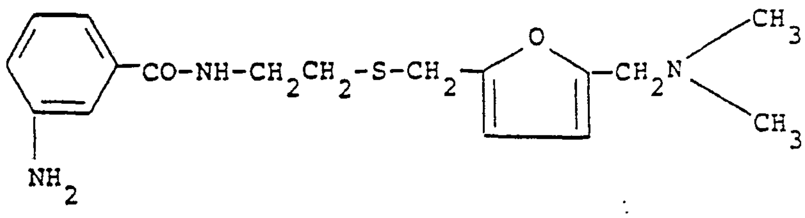

avec un composé de formule

$$Y = C = Z$$

dans laquelle Y représente deux groupes méthylthio et Z représente un groupe N—CN ou CH—$NO_2$, ou bien Y représente un atome d'oxygène ou de soufre et Z représente un groupe N—R, où R a la même signification donnée ci-dessus, dans un solvant organique à une température comprise entre 20 °C et la température d'ébullition du solvant employé et, lorsque Y représente deux groupes méthylthio et Z représente un groupe N—CN ou CH—$NO_2$, on traite le produit résultant avec une amine de formule R—$NH_2$, où R est tel que défini ci-dessus, dans un solvant organique ; et on transforme éventuellement le produit ainsi obtenu dans ses sels pharmaceutiquement acceptables.

2. Procédé selon la revendication 1, caractérisé en ce que le produit de départ, 3-amino-N-[2-(5-diméthylaminométhylfuran-2-ylméthylthio)éthyl] benzamide, ou un de ses sels, est préparé par réduction du 3-nitro-N-[2-(5-diméthylaminométhylfuran-2-ylméthylthio)éthyl]benzamide en utilisant, comme agent réducteur, l'hydrogène naissant de la réaction entre fer et acide chlorhydrique.

**Claims** (for the Contracting States : BE, CH, DE, FR, GB, IT, LI, LU, NL, SE)

1. Benzamides of formula :

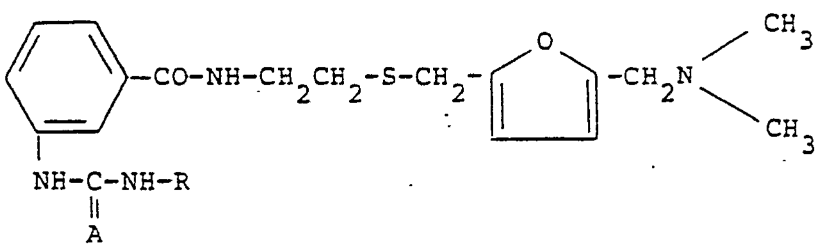

wherein A represents an oxygen or sulfur atom, a N—CN or CH—$NO_2$ group and R represents an alkyl group of from 1 to 6 carbon atoms, or an optionally substituted phenyl, pyridyl or pyridyl 1-oxide group ; as well as the pharmaceutically acceptable addition salts thereof.

2. The 3-(3-methylthioureido)-N-[2-(5-dimethylaminomethylfuran-2-ylmethylthio)ethyl]benzamide or the pharmaceutically acceptable salts thereof.

3. A process for the preparation of a compound of formula

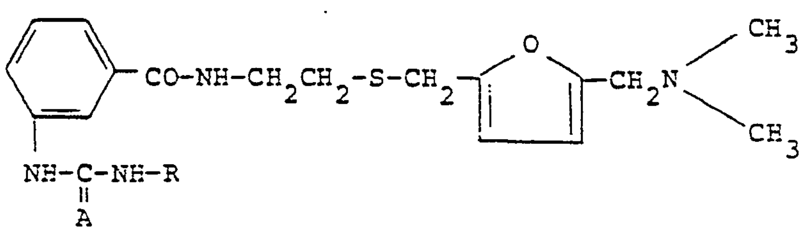

wherein A represents an oxygen or sulfur atom, a N—CN or CH—$NO_2$ group and R represents an alkyl group of from 1 to 6 carbon atoms, or an optionally substituted phenyl, pyridyl or pyridyl 1-oxide group, and of their pharmaceutically acceptable salts, characterized in that it comprises reacting the 3-amino-N-[2-(5-dimethylaminomethylfuran-2-ylmethylthio)-ethyl]benzamide of formula

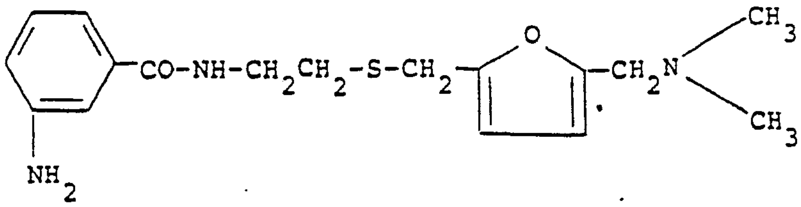

with a compound of formula

$$Y = C = Z$$

wherein Y represents two methylthio groups and Z represents a N—CN or CH—$NO_2$ group or Y represents an oxygen or sulfur atom and Z represents a N—R group, where R has the above stated meaning in an organic solvent at a temperature between 20 °C and the boiling temperature of the solvent employed and, when Y represents two methylthio groups and Z represents a N—CN or CH—$NO_2$ group, the resulting product is treated with an amine of formula R—$NH_2$ wherein R is as defined hereinabove, in an organic solvent ; and optionally converting the compound thus obtained into the pharmaceutically salts thereof.

4. A process according to claim 3, characterized in that the starting product, the 3-amino-N-[2-(5-dimethylaminomethylfuran-2-yl-methylthio)ethyl]benzamide or a salt thereof, is prepared by reducing the 3-nitro-N-[2-(5-dimetylaminomethylfuran-2-ylmethylthio)ethyl]benzamide, utilizing hydrogen nascent from the reaction of iron and hydrochloric acid as a reducing agent.

5. The 3-amino-N-[2-(5-dimethylaminomethylfuran-2-yl-methylthio)ethyl]benzamide of formula

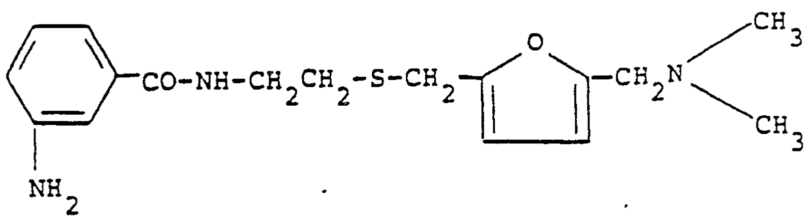

or the acid additión salts thereof.

6. A pharmaceutical composition containing as active ingredient a compound according to one of claims 1 and 2.

7. A pharmaceutical composition according to claim 6, in dosage unit form, containing from 10 to 1 000 mg of active ingredient, per dosage unit, in admixture with a pharmaceutical carrier.

8. A pharmaceutical composition according to claim 7, containing from 100 to 500 mg of active ingredient per dosage unit.

**Claims** (for the Contracting State AT)

1. Process for preparing a compound of formula :

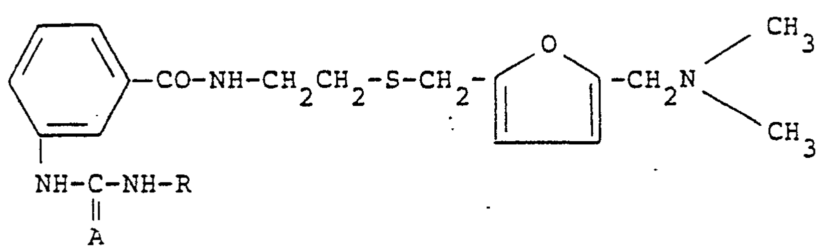

wherein A represents an oxygen or sulfur atom, a N—CN or CH—$NO_2$ group and R represents an alkyl group of from 1 to 6 carbon atoms, or an optionally substituted phenyl, pyridyl or pyridyl 1-oxide group ; and of their pharmaceutically acceptable salts, characterized in that it comprises reacting the 3-amino-N-[2-(5-dimethylaminomethylfuran-2-ylmethylthio)-ethyl]benzamide of formula

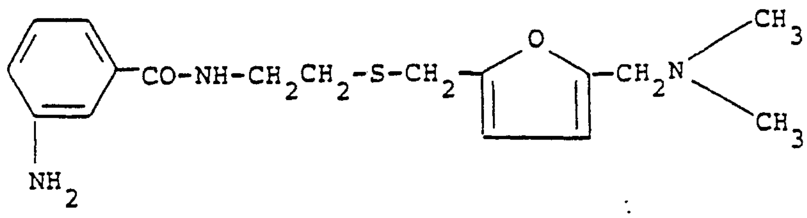

with a compound of formula

$$Y = C = Z$$

wherein Y represents two methylthio groups and Z represents a N—CN or CH—$NO_2$ group or Y represents

an oxygen or sulfur atom and Z represents a N—R group, where R has the above stated meaning in an organic solvent at a temperature between 20 °C and the boiling temperature of the solvent employed and, when Y represents two methylthio groups and Z represents a N—CN or CH—$NO_2$ group, the resulting product is treated with an amine of formula R—$NH_2$ wherein R is as defined hereinabove, in an organic solvent ; and optionally converting the compound thus obtained into a pharmaceutically acid addition salt thereof.

2. A process according to claim 1, characterized in that the starting product, the 3-amino-N-[2-(5-dimethylaminomethylfuran-2-ylmethylthio)ethyl]benzamide or a salt thereof, is prepared by reducing the 3-nitro-N-[2-(5-dimetylaminomethylfuran-2-ylmethylthio)ethyl]benzamide, utilizing hydrogen nascent from the reaction of iron and hydrochloric acid as a reducing agent.

**Patentansprüche** (für die Vertragsstaaten : BE, CH, DE, FR, GB, IT, LI, LU, NL, SE)

1. Benzamide der Formel

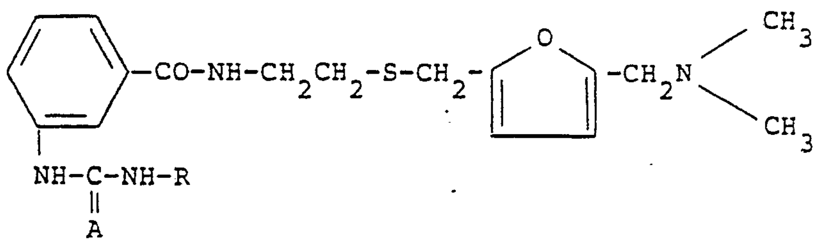

worin A ein Sauerstoff- oder Schwefelatom, eine Gruppe N—CN oder CH—$NO_2$ darstellt und R eine 1 bis 6 Kohlenstoffatome enthaltende Alkylgruppe, eine Phenylgruppe, eine Pyridylgruppe oder eine Pyridyl-1-oxidgruppe, die gegebenenfalls substituiert sind, bedeutet ; sowie ihre pharmazeutisch akzeptablen Salze.

2. 3-(3-Methylthioureido)-N-[2-(5-dimethylaminomethylfuran-2-yl-methylthio)-äthyl]-benzamid und seine pharmazeutisch akzeptablen Salze.

3. Verfahren zur Herstellung einer Verbindung der Formel

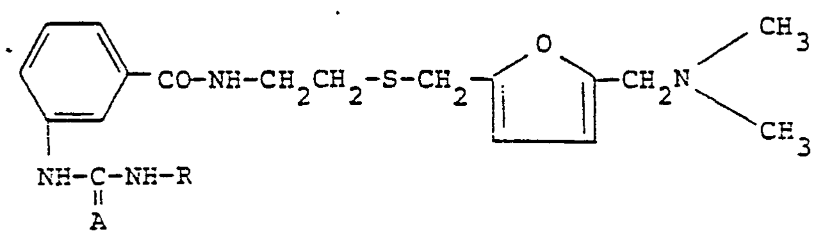

worin A ein Sauerstoff- oder Schwefelatom, eine Gruppe N—CN oder CH—$NO_2$ darstellt und R eine 1 bis 6 Kohlenstoffatome enthaltende Alkylgruppe, eine Phenylgruppe, eine Pyridylgruppe oder eine Pyridyl-1-oxidgruppe, die gegebenenfalls substituiert sind, bedeutet, und ihrer pharmazeutisch akzeptable Salze, dadurch gekennzeichnet, daß man 3-Amino-N-[2-(5-dimethylaminomethylfuran-2-yl-methylthio)-äthyl]-benzamid der Formel

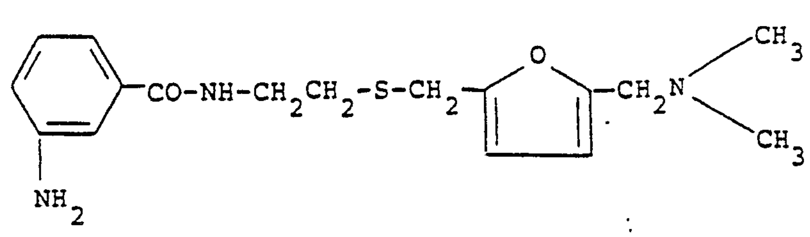

mit einer Verbindung der Formel

$$Y = C = Z$$

worin Y zwei Methylthiogruppen bedeutet und Z für eine Gruppe N—CN oder CH—$NO_2$ steht, oder aber Y ein Sauerstoff- oder Schwefelatom darstellt und Z für eine Gruppe N—R, worin R obige Bedeutung hat, steht, in einem organischen Lösungsmittel bei einer Temperatur zwischen 20 °C und der Kochtemperatur des verwendeten Lösungsmittels behandelt und man das erhaltene Produkt, wenn Y zwei Methylthiogruppen bedeutet und Z für eine Gruppe N—CN oder CH—$NO_2$ steht, mit einem Amin der Formel

R—$NH_2$, worin R obige Bedeutung hat, in einem organischen Lösungsmittel behandelt ; und man gegebenenfalls das so erhaltene Produkt in seine pharmazeutisch akzeptablen Salze umwandelt.

4. Verfahren nach Anspruch 3, dadurch gekennzeichnet, daß das Ausgangsprodukt 3-Amino-N-[2-(5-dimethylaminomethylfuran-2-yl-methylthio)-äthyl]-benzamid oder eines seiner Salze hergestellt wird durch Reduktion von 3-Nitro-N-[2-(5-dimethylaminomethylfuran-2-yl-methylthio)-äthyl]-benzamid unter Verwendung von Wasserstoff, der durch die Reaktion zwischen Eisen und Salzsäure entsteht, als Reduktionsmittel.

5. 3-Amino-N-[2-(5-dimethylaminomethylfuran-2-yl-methylthio)-äthyl]-benzamid der Formel

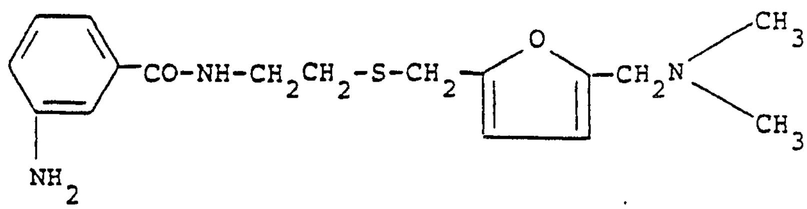

und seine Säureadditionssalze.

6. Pharmazeutische Zusammensetzung, die als Wirkstoff eine Verbindung nach einem der Ansprüche 1 und 2 enthält.

7. Pharmazeutische Zusammensetzung nach Anspruch 6 in Dosiseinheitsform, worin jede Dosiseinheit 10 bis 1 000 mg Wirkstoff in Mischung mit einem pharmazeutischen Exzipienten enthält.

8. Pharmazeutische Zusammensetzung nach Anspruch 7, die 100 bis 500 mg Wirkstoff pro Dosiseinheit enthält.

**Patentansprüche** (für den Vertragsstaat AT)

1. Verfahren zur Herstellung einer Verbindung der Formel

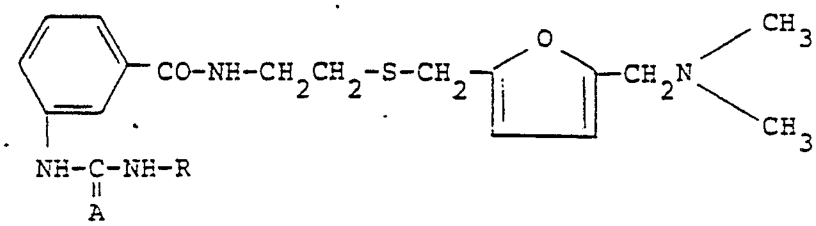

worin A ein Sauerstoff- oder Schwefelatom, eine Gruppe N—CN oder CH—$NO_2$ darstellt und R eine 1 bis 6 Kohlenstoffatome enthaltende Alkylgruppe, eine Phenylgruppe, eine Pyridylgruppe oder eine Pyridyl-1-oxidgruppe, die gegebenenfalls substituiert sind, bedeutet, und ihrer pharmazeutisch akzeptable Salze, dadurch gekennzeichnet, daß man 3-Amino-N-[2-(5-dimethylaminomethylfuran-2-yl-methylthio)-äthyl]-benzamid der Formel

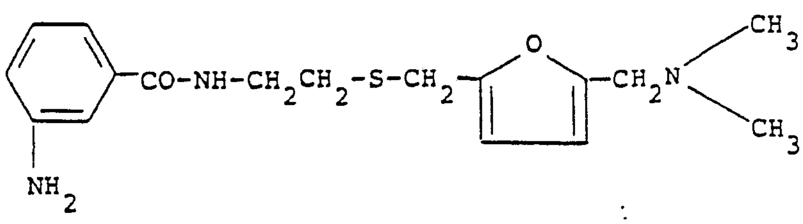

mit einer Verbindung der Formel

$$Y = C = Z$$

worin Y zwei Methylthiogruppen bedeutet und Z für eine Gruppe N—CN oder CH—$NO_2$ steht, oder aber Y ein Sauerstoff- oder Schwefelatom darstellt und Z für eine Gruppe N—R, worin R obige Bedeutung hat, steht, in einem organischen Lösungsmittel bei einer Temperatur zwischen 20 °C und der Kochtemperatur des verwendeten Lösungsmittels behandelt und man das erhaltene Produkt, wenn Y zwei Methylthiogruppen bedeutet und Z für eine Gruppe N—CN oder CH—$NO_2$ steht, mit einem Amin der Formel R—$NH_2$, worin R obige Bedeutung hat, in einem organischen Lösungsmittel behandelt ; und man gegebenenfalls das so erhaltene Produkt in seine pharmazeutisch akzeptablen Salze umwandelt.

2. Verfahren nach Anspruch 1, dadurch gekennzeichnet, daß das Ausgangsprodukt 3-Amino-N-[2-(5-dimethylaminomethylfuran-2-yl-methylthio)-äthyl]-benzamid oder eines seiner Salze hergestellt wird durch Reduktion von 3-Nitro-N-[2-(5-dimethylaminomethylfuran-2-yl-methylthio)-äthyl]-benzamid unter Verwendung von Wasserstoff, der durch die Reaktion zwischen Eisen und Salzsäure entsteht, als Reduktionsmittel.